# EUROPEAN PATENT APPLICATION

(11) **EP 0 645 148 A1**
(43) Date of publication of application: **29.03.1995**
(21) Application number: 94306944.3
(22) Date of filing: 22.09.1994
(51) Int. Cl.: A61L 9/12, F24F 3/12

(54) **Photoelectric air conditioning device**

(30) Priority: 23.09.1993 GB 2033493
(71) Applicant: Dudley Industries Ltd., Lytham, Lancs. FY8 5AU (GB)
(72) Inventor: Fielding, Robert Michael George, Lytham-St. Annes, Lancashire (GB); Brown, Keith, Lytham-St. Annes, Lancashire (GB)
(74) Representative: Powell, Stephen David

(57) **Abstract**

A housing (10) contains a perfume cassette (30) and a fan (21), a photoelectric cell (14) supplying a capacitor (41) that is periodically discharged to drive the fan. The device includes a cycle counter (57) which activates a warning device (58) when casette (30) reaches the end of its expected operating life.

## Description

The present invention relates to air conditioning devices, and in particular to air freshening devices powered by photoelectricity.

Air freshening devices are used in the home and workplace to eliminate unpleasant odours. It is desirable that such devices should be portable for ease of installation. However, if the device is to be effective some form of fan is needed which requires a power source. Battery power could be used but the batteries would require periodic replacement.

US-A-5,105,133 discloses a battery-powered automatic perfumer which can operate in a continuous mode, an intermittent mode in which operation is controlled by a timing circuit, or a light-activated mode in which the timing circuit is disabled until a photoelectric element detects a predetermined intensity of light.

GB-A-2 247 623 discloses an air freshening device comprising a housing having an air flow path therethrough, fan means to move air along the flow path, means to distribute an air freshening substance into the moving air, photoelectric power means to drive the fan means, and means to control the operation of the device.

The means to control the operation of the device comprises a standard timing circuit. The term timing circuit is to be taken to mean a circuit incorporating a clock component. This requires a continuous power supply in the form of a rechargeable battery. The provision of such a battery is expensive, especially as the lifetime of rechargeable batteries is finite.

Accordingly, the present invention comprises an air freshening device comprising a housing having an air flow path therethrough, fan means to move air along the flow path, means to distribute an air freshening substance into the moving air, photoelectric power means to drive the fan means, and means to control the operation of the device, wherein the control means comprises capacitor means that is charged by the photoelectric power means and discharges to drive the fan means when the voltage across it reaches a predetermined upper value and recharges when the voltage reaches a predetermined lower value.

In a preferred arrangement, the capacitor means is connected to a plurality of transistor switches which allow the capacitor means to charge until the voltage across it reaches said predetermined upper value; the charge in the capacitor means is then discharged until the voltage reaches said predetermined lower value. Preferably, both of said predetermined values can be varied. This may be achieved by providing a variable resistance between the capacitor means and one or more of the transistor switches.

Preferably, the device further comprises timing means which actuate operator warning means when the useful life of the means to distribute an air freshening substance has expired. The timing means may comprise a timing circuit and the operator warning means an LED.

Preferably, the timing means is powered by the photoelectric power means.

Preferably, the device can operate from artificial light. The light may be from fluorescent lights. The minimum operable light level may be 70 Lux.

In order that the invention and its various other features may be understood more easily, preferred embodiments thereof will now be described by way of example only, with reference to the drawings, wherein:
Fig. 1 is a vertical section through the device;
Fig. 2 is a circuit diagram of the electronic circuit incorporated in the device; and
Fig. 3 is an alternative circuit diagram for the device.

The air freshening device comprises a housing 10, a fan 20, a perfume cassette 30 and an electric circuit 40. The device is mounted on a vertical wall 50.

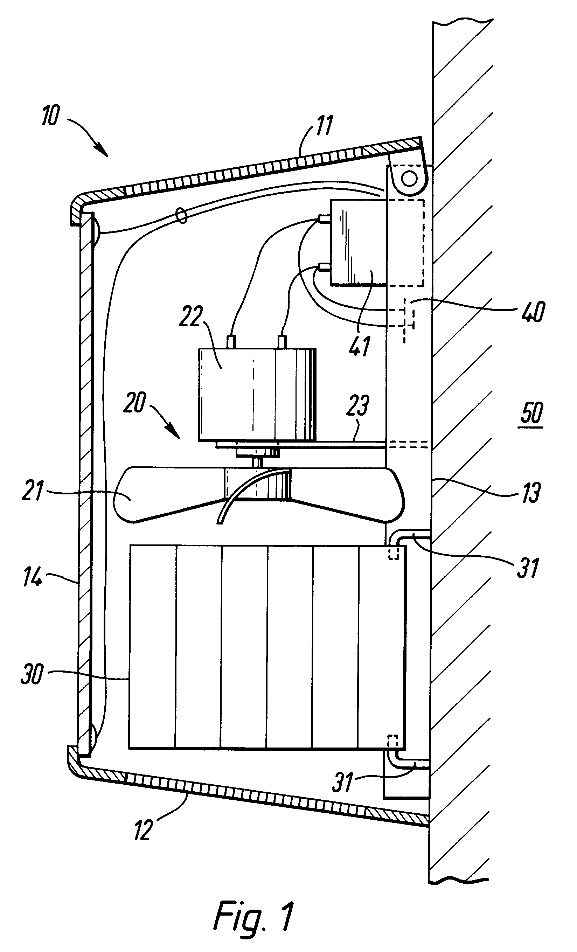

The housing 10 has a perforated lid 11 and base 12. The housing is hinged to allow access to the inside of the unit. The back panel 13 of the housing and the side panels (not shown in Fig. 1) are solid. Means (not shown) are provided to fix the back panel to the wall 50. The front panel 14 is a photoelectric panel comprising one or more photoelectric cells.

The fan 20 comprises a propeller 21 mounted on the shaft of a miniature electric motor 22. The motor is supported by a bracket 23 fixed to the back panel 13 of the housing 10.

The perfume cassette 30 is a solid block of perfume material having vertical channels (not shown) through it. The cassette is detachably mounted on the back panel 13 of the housing 10 by brackets 31.

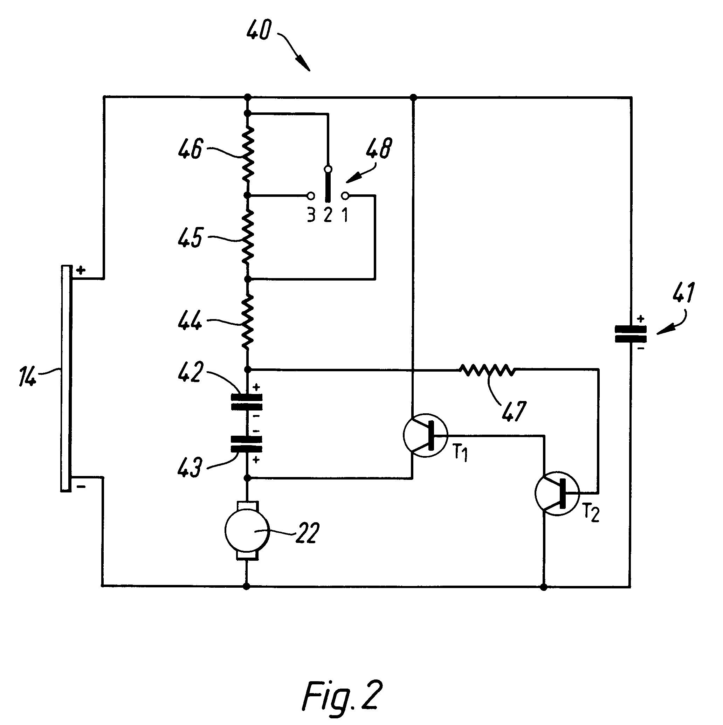

The electric circuit 40 in Fig. 2 is connected to the photoelectric panel 14. The circuit contains a high value capacitor 41 which is charged by the photoelectric panel 14. The input to and output from the capacitor are governed by two transistor switches T₁ and T₂. Back to back capacitors 42,43 improve the efficiency of the circuit. Typical values are 100µF.

The photoelectric panel 14 is a multi elemental photoelectric cell which has an output between 2.5 V at 0.04 mA, and 6.5 V at 0.7 mA. The output from the cell is stored in the capacitor 41 (typically 16 V and 10000 µF).

Resistors 44-47 tune the operating characteristics of the device to different light levels. Switch 48 allows a different resistance to be applied. Typical values for resistors 44 to 46 are 20 MΩ and resistor 47 is 120 kΩ. Switch position 2 is for light levels 70-400 Lux, position 3 for 400-600 Lux, and position 1 for levels in excess of 600 Lux. At lower light levels the revolution rate of the motor decreases.

When the voltage across the capacitor 41 reaches a predetermined upper level the transistor switches T₁ and T₂ discharge the capacitor 41 to the motor 22, thereby driving the fan 21. This expels perfume from the device. When the voltage falls to a predetermined lower level the transistor switches reset and the capacitor 41 is again charged by the cells of the photoelectric panel 14.

The operation of the device continues in this manner in a cyclic fashion, releasing perfume about every three minutes even at low light levels.

Unlike the known device the present invention does not require a rechargeable battery and does not use a timing circuit for activating the fan.

The device can operate in a wide range of light conditions whilst still releasing perfume every three minutes.

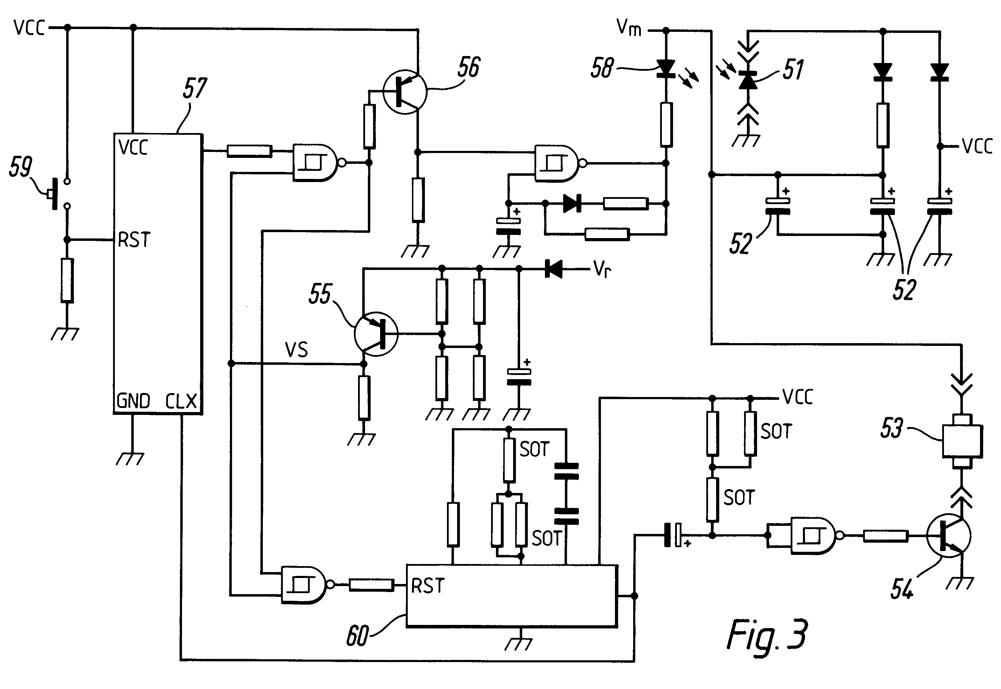

Fig. 3 shows an alternative circuit comprising a photoelectric cell 51, three high value capacitors 52 for storing charge from the cell and a motor 53 for driving the fan in the device. The circuit contains three transistors 54, 55, 56.

A further feature of the circuit in Fig. 3 is a cycle counter 57 which is connected to an LED 58. The counter activates the LED when the useful life of the perfume cassette 30 has expired, usually after about one month's operation. When the operator fits a new cassette into the device the clock is reset using button 59.

The motor 53 is connected to the capacitors 52 and a transistor 54. When the voltage across the capacitors reaches its upper predetermined level the transistor 54 connects the motor to ground thereby powering the fan. Every time the transistor 54 is switched, a signal is sent to an integrated circuit 60. At each cycle the circuit 60 sends a pulse to the cycle counter 57. The counter activates the LED after a predetermined number of signals are received. Integrated circuit 60 also controls the cycle time.

The part of the circuit in Fig. 3 around the transistor 55 is to turn off the cycle counter 57 at low light levels. Typically, the counter is turned back on when the value of Vᵣ (the voltage from photelectric cell 51) increases to above 4.2 to 4.5 V. Typically, the motor voltage Vₘ = Vᵣ - 0.7 V.

The circuit in Fig. 3 does not require rechargeable batteries. Instead the cycle counter 57 is powered by the photoelectric cell 51.

The circuit shown in Fig. 3 reminds the operator that the device needs attention, thereby ensuring that the air in the washroom is kept fresh.

A number of modifications can be made to the device. Two or more motors and fans can be used in a single device.

The photoelectric panel 14 can be positioned away from the body of the device to prevent vandals reaching it. An ioniser unit can be incorporated in the device.

The device may be used to dispense insecticides or any substances in vapour form.

## Claims

1. An air conditioning device comprising a housing (10) having an air flow path therethrough, fan means (20) to move air along the flow path, means (30) to distribute an air conditioning substance into the moving air, photoelectric power means (14) to drive the fan means, and means (40) to control the operation of the device, characterised in that the control means comprises capacitor means (41) that is charged by the photoelectric power means and discharges to drive the fan means when the voltage across it reaches a predetermined upper value and recharges when the voltage reaches a predetermined lower value.

2. A device according to claim 1, wherein the capacitor means (41) is connected to a plurality of transistor switches (T₁, T₂) which allow the capacitor means to charge until the voltage across it reaches said predetermined upper value, the charge in the capacitor then being discharged until the voltage reaches said predetermined lower value.

3. A device according to claim 2, comprising means (44-48) for adjusting the predetermined upper and lower values of the voltage.

4. A device according to claim 3, wherein the adjusting means comprises a variable resistance arrangement (44-48) between the capacitor means and one or more of the transistor switches.

5. A device according to any preceding claim, further comprising means (57) which actuate operator warning means (58) when the useful life of the means (30) to distribute an air conditioning substance has expired.

6. A device according to claim 5, wherein the means which actuate operator warning means comprise counter means (57).

7. A device according to claim 6, wherein the counter means (57) include reset means (59).

8. A device according to any of claims 5 to 7, wherein the operator warning means comprises an LED (58).

9. A device according to any of claims 5 to 8 wherein the means (57) which actuate operator warning means are powered by the photoelectric power means (51).
